# EUROPEAN PATENT SPECIFICATION

(11) **EP 1 161 521 B1**
(45) Date of publication and mention of the grant of the patent: **16.04.2003**
(21) Application number: 00905194.7
(22) Date of filing: 23.02.2000
(51) Int. Cl.: C12N 5/06, A61P 25/28

(54) **TRANSPLANTATION OF HAEMATOPOIETIC CELLS**
TRANSPLANTATION VON HÄMATOPOIETISCHEN ZELLEN
TRANSPLANTATION DE CELLULES HEMATOPOIETIQUES

(30) Priority: 24.02.1999 GB 9904281
(43) Date of publication of application: 12.12.2001
(73) Proprietor: Reneuron Limited, Guildford, Surrey GU2 7AF (GB)
(72) Inventor: PRICE, Jack, Guildford, Surrey GU2 7AF (GB)
(74) Representative: Perry, Robert Edward
(86) International application number: GB0000636
(87) International publication number: WO00050568

(56) References cited:
- WO-A-92/11355
- WO-A-93/18137
- WO-A-97/10329
- WO-A-98/07841
- US-A- 5 817 773
- J.A. ALLAY ET AL.: "LACZ AND INTERLEUKIN-3 EXPRESSION IN VIVO AFTER RETROVIRAL TRANSDUCTION OF MARROW-DERIVED HUMAN OSTEOGENIC MESENCHYMAL PROGENITORS" HUMAN GENE THERAPY, vol. 8, no. 12, 10 August 1997 (1997-08-10), pages 1417-1427, XP002145778
- M. CHOPP ET AL.: "ADULT BONE MARROW TRANSPLANTATION FOR TREATMENT OF STROKE IN ADULT MICE" SOCIETY FOR NEUROSCIENCE, vol. 25, 1999, page 528.2 XP000900788 cited in the application
- HODGES, HELEN (1) ET AL: "Cognitive deficits induced by global cerebral ischaemia: Prospects for transplant therapy." PHARMACOLOGY BIOCHEMISTRY AND BEHAVIOR, (1997) VOL. 56, NO. 4, PP. 763-780., vol. 56, no. 4, 1997, pages 763-780, XP000857876 cited in the application
- Transcript based on a presentation at the British Society for Immunology Annual Meeting. Harrogate, UK, 3rd and 4th December 1998. Stem / Progenitor Cells. Hans-Jörg Bühring

## Description

### Field of the Invention

The present invention relates to the correction of sensory, motor and/or cognitive deficits by the intracerebral transplantation of cells, and medicaments therefor.

### Background to the Invention

Sensory, motor and/or cognitive deficits are caused by many diseases and may also be caused when the brain undergoes trauma. For example, motor dysfunction is one symptom of Parkinson's disease. As yet, in most cases, there is no satisfactory treatment available.

Bjornson *et al*, Science (1999) 283:534-537, describes the ability of neural stem cells to produce a variety of blood cell types, including myeloid, lymphoid and haematopoietic cells. It is believed that the neural stem cells contain appropriate mechanisms required to express otherwise silent genetic information to respond to signals that normally stimulate blood stem cells. The experiment suggests that the adult blood system contains powerful signals that "activate" the neural stem cells.

Haematopoietic cells are the progenitor cells for all of the blood cells, including leukocytes and erythrocytes. The transplantation of such cells has been proposed, for example, in WO-A-92/11355, in connection with haematopoietic disorders, including sickle cell anaemia and haemophilia. Methods for culturing and genetically altering haematopoietic stem cells are disclosed in WO-A-92/11355 and WO-A-93/18137.

### Summary of the Invention

The present invention is based in part on the observation that, when transplanted into a damaged or diseased brain, haematopoietic stem cells appear to respond to signals from the damaged or diseased brain by taking up a phenotype that is able to replace or compensate for functional deficits to which the damage or disease otherwise leads.

For use in the present invention, the haematopoietic stem cells should be capable of differentiating into cells appropriate to repair or compensate for the damage or disease in the target area of the brain. It will be appreciated that cells useful for transplantation need not be capable of differentiation into all types or phenotypes of neural cells.

The present invention may be used in the treatment of any mammal but is especially useful in the context of treatment of humans, especially treatment with human cells and cell lines.

The cells used in the present invention are capable of correcting a sensory, motor and/or cognitive deficit when implanted into a damaged part of the human brain. The term "damage" used herein includes reduction or loss of function. The term also includes cell loss. Damage may be caused by any of a variety of means including physical trauma, hypoxia (lack of oxygen), chemical agents, for example, damage may be caused by drug abuse, and disease. The following diseases and pathological conditions are examples of diseases or conditions which result in sensory, motor and/or cognitive deficits which may be treated in accordance with the present invention: traumatic brain injury, stroke, perinatal ischaemia, including cerebral palsy, Alzheimer's, Pick's and related dementing neurodegenerative diseases, multi-infarct dementia, Parkinson's and Parkinson's type diseases, Huntington's disease, Korsakoff's disease and Creuzfeld-Jacob disease. Amnesia, particularly following transitory global ischaemia such as after cardiac arrest or coronary bypass surgery, may also be treated in accordance with the present invention.

The cells may also be administered to sites distant from the actual site of damage, for example, the cells may be administered to the contra-lateral region from that exhibiting damage.

The present invention provides for the use of haematopoietic stem cells, which are not human embryonic stem cells, optionally in isolated form, in the manufacture of a medicament for intracerebral transplantation into a domaged brain for the treatment of a sensory, motor and/or cognitive deficit. The medicament to be administered comprises haematopoietic stem cells.

The present invention further provides for the use of conditionally immortal, haematopoietic stem cells in the manufacture of a medicament for the treatment of a sensory, motor and/or cognitive deficit. The medicament to be administered comprises conditionally immortal, haematopoietic stem cells.

The conditionally immortal cells used in the present invention may be from clonal cell lines or may be of mixed population. Cells from clonal cell lines may be preferred. Cells from a single cell line may be used or a mixture of cells from two or more cell lines may be used.

### Description of the Invention

The present invention is based on the realisation that when haematopoietic stem cells are implanted into a damaged brain, the cells surprisingly differentiate into a form of cell that is capable of repairing the damage and improve function. The phenotype of the differentiated cells may be the same as the phenotype of the damaged or lost cells, however, the differentiated cells may be of a different phenotype, or of a number of phenotypes. In any case, the cells take up a phenotype that is capable of functionally integrating and compensating for the damaged or lost cells. That is assisted by the propensity, that we have discovered, of the cells to migrate to, and seek out, damaged tissue.

The use of stem cells means that with one clonal cell line it is possible to repair damage in a number of different areas of the brain. It also means that if more than one particular cell type is required to repair damage in a given area then a single cell line will be capable of differentiating into the different types of cells required.

Conditionally immortal cells are cells which are immortal under certain permissive conditions but are not immortal under nonpermissive conditions. In the present case this means that by conditionally immortalising the stem cells and maintaining them under permissive conditions the development of the stem cells may be arrested at a chosen stage and they may be propagated for long periods. Use of conditionally immortalisation allows the development of clonal lines which are readily expandable in vitro. If the conditions under which the cells are maintained are switched to nonpermissive-conditions, the development of the cells is allowed to continue. If the correct conditions are provided the cells will continue to develop and will differentiate.

Immortalised cells are usually prepared by the transduction of an oncogene into cells. There is therefore a risk of tumour formation in the long term, so such cells are not preferred for use in the present invention.

Conditionally immortal cells have the advantages of immortal cells in that they are "frozen" in the desired stage of development, are easily maintained and multiply well when under permissive conditions but they may be used in transplants as long as the environment into which they are transplanted has nonpermissive conditions. In the case of the cells of the present invention the gene used to confer conditional immortality should be chosen so that the conditions present in the brain will correspond to nonpermissive conditions.

An example of a suitable conditionally immortal oncogene is that which expresses the non-DNA binding, temperature-sensitive T antigen; a description of which may be found in US-A-5688692 or WO-A-97/10329.

If non-immortal cells are used then these may be maintained *in vitro* in culture media with the addition of growth factors as disclosed in WO-A-92/11355 and WO-A-93/18137.

The gene which is used to confer conditional immortality may be incorporated into cells after extraction from the bone marrow of an animal.

The cells used in the treatment of humans should preferably be derived from human cells to reduce problems with immune rejection. This requires the extraction of cells from the bone marrow of a human.

However, the cells do not necessarily have to be conditionally immortal, and may be obtained directly from the patient to be treated.

To treat a patient it is generally of assistance to know where damage has occurred in the brain. Once the existence of damage has been established, whether it be in one isolated area or in several areas, treatment by implantation of cells into the damaged area may be carried out. In many cases, however, the location and/or type of damaged tissue may be unknown or only poorly characterised. For example, neurodegenerative diseases may lead to widespread damage to different types of cells. Treatment of such damage is still possible and is assisted by the ability of the haematopoietic stem cells to migrate extensively once transplanted and to seek out damaged tissue. The stem cells may be transplanted at a single site, or preferably at multiple sites, and may be able to migrate to the site(s) of damage and, once there, differentiate in response to the local microenvironment, into the necessary phenotype or phenotypes to improve or restore function.

After treatment the progress of the patient may be monitored using sensory, motor and/or cognitive tests and/or, if desired, tests which monitor brain activity in selected areas of the brain. For example, tests for cognitive function may be performed before and after transplantation.

Preferably, treatment will substantially correct a sensory, motor and/or cognitive deficit (behavioural and/or psychological deficits). However, that may not always be possible. Treatment according to the present invention and with the cells, medicaments and pharmaceutical preparations of the invention, may lead to improvement in function without complete correction. Such improvement will be worthwhile and of value.

The number of cells to be used will vary depending on the nature and extent of the damaged tissue. Typically, the number of cells used in transplantation will be in the range of about one hundred thousand to several million. Treatment need not be restricted to a single transplant. Additional transplants may be carried out to further improve function.

To study the transplantation in animal models the tests described in Hodges, *et al*, Pharmacology, Biochemistry and Behaviour (1997) 56(4):763-780 may be used. One test utilises rats in which the technique for four-vessel occlusion (4 VO), simulating human heart attack, causes relatively circumscribed and specific damage to the CA 1 pyramidal cells of the dorsal hippocampus, along with a cognitive deficit manifest as difficulty in locating a submerged and invisible platform in a swimming pool. This provides a model of cognitive dysfunction occurring as a consequence of a common form of brain damage, i.e., transient loss of blood supply to the brain, for example, as may occur during cardiac arrest.

Methods for transplantation of cells into humans and animals are known to those in the art and are described in the literature in the art. The term "transplantation" used herein includes the transplantation of cells which have been grown *in vitro*, and may have been genetically modified, as well as the transplantation of material extracted from another organism. Cells may be transplanted by implantation by means of microsyringe infusion of a known quantity of cells in the target area where they would normally disperse around the injection site. They may also be implanted into ventricular spaces in the brain. If implanted into the neonate then they may disperse throughout the entire brain.

The phrase "intracerebral transplantation" used herein includes transplantation into any portion of the brain. Transplantation is not restricted to the front and larger part of the brain.

The utility of the present invention has now been reported independently in Chopp *et al*, Society for Neuroscience (1999), Vol. 25, Abstract No. 528.2. This Abstract was published after the priority date of the present Application and details the intracerebral transplantation of haematopoietic stem cells in mice.

As detailed in the Abstract, adult mice were subjected to middle cerebral artery occlusion (MCAo), and cultured haematopoietic stem cells (infused with growth factor) were transplanted into the ischaemic striatum (4 days after MCAo). Cell type specific markers were used for identification of the donor cells on transplantation. The results showed that donor cells survived transplantation and were morphologically detectable in the ischaemic striatum, exhibiting the phenotype of neuronal and astrocyte cells.

Suitable excipients, diluents and carriers will be apparent to the-skilled person and formulations suitable for intracerebral transplantation will also be apparent.

The stem cells useful in the present invention may be genetically transformed to express a heterologous gene product. In particular, a therapeutic gene product that exerts its effect at the site of damage. Examples of suitable gene products are disclosed in US-A-5958767.

## Claims

1. Use of a haematopoietic stem cell, which is not a human embryonic stem cell, in the manufacture of a medicament for intracerebral transplantation into a damaged brain for the treatment of a sensory, motor and/or cognitive deficit.

2. Use according to claim 1, wherein the treatment is for Alzheimer's disease, Parkinson's disease, Korsakoff's disease or Creuzfeld-Jacob disease.

3. Use according to claim 1 or claim 2, wherein the haematopoietic stem cell is conditionally immortal.

4. Use according to claim 3, wherein the cell comprises a temperature-sensitive oncogene which is not expressed at a temperature above 35°C.

5. Use according to claim 4, wherein the oncogene expresses the SV40 T-antigen.

6. Use according to any preceding claim, wherein the haematopoietic stem cell is genetically transformed to express a therapeutic heterologous gene product.

## Patentansprüche

1. Verwendung einer haematopoetischen Stammzelle, bei der es sich nicht um eine menschliche embryonale Stammzelle handelt, bei der Herstellung eines Arzneimittels für die intrazerebrale Transplantation in ein geschädigtes Gehirn zur Behandlung eines sensorischen, motorischen und/oder kognitiven Defizits.

2. Verwendung nach Anspruch 1, wobei die Behandlung Alzheimer'sche Krankheit, Parkinson'sche Krankheit, Korsakoff'sche Krankheit oder Creutzfeld-Jacob-Krankheit betrifft.

3. Verwendung nach Anspruch 1 oder Anspruch 2, wobei die haematopoetische Stammzelle konditional unsterblich ist.

4. Verwendung nach Anspruch 3, wobei die Zelle ein temperaturempfindliches Onkogen umfasst, das bei einer Temperatur oberhalb 35°C nicht exprimiert wird.

5. Verwendung nach Anspruch 4, wobei das Onkogen das SV40 T-Antigen exprimiert.

6. Verwendung nach einem der vorstehenden Ansprüche, wobei die haematopoetische Stammzelle genetisch transformiert ist, um ein therapeutisches heterologes Genprodukt zu exprimieren.

## Revendications

1. Utilisation d'une cellule-souche hématopoïétique qui n'est pas une cellule-souche d'embryon humain dans la fabrication d'un médicament pour transplantation intracérébrale dans un cerveau endommagé en vue du traitement d'un déficit sensoriel, moteur et/ou cognitif.

2. Utilisation selon la revendication 1, dans laquelle le traitement est celui de la maladie d'Alzheimer, de la maladie de Parkinson, de la maladie de Korsakoff ou de la maladie de Kreuzeld-Jacob.

3. Utilisation selon la revendication 1 ou la revendication 2, dans laquelle la cellule-souche hématopoïétique est conditionnellement immortelle.

4. Utilisation selon la revendication 3, dans laquelle la cellule comprend un oncogène thermosensible qui n'est pas exprimé à une température supérieure à 35°C.

5. Utilisation selon la revendication 4, dans laquelle l'oncogène exprime l'antigène SV40-T.

6. Utilisation selon l'une quelconque des revendications précédentes, dans laquelle la cellule-souche hématopoïétique est transformée génétiquement pour exprimer une produit constitué d'un gène hétérologue thérapeutique.
